# EUROPÄISCHE PATENTSCHRIFT

(11) **EP 3 620 196 B1**
(45) Veröffentlichungstag und Bekanntmachung des Hinweises auf die Patenterteilung: **07.06.2023**
(21) Anmeldenummer: 19192229.3
(22) Anmeldetag: 19.08.2019
(51) Int. Cl.: A61M 16/04

(54) **BEATMUNGSVORRICHTUNG**
VENTILATION APPARATUS
DISPOSITIF DE RESPIRATION

(30) Priorität: 28.08.2018 DE 102018121001
(43) Veröffentlichungstag der Anmeldung: 11.03.2020
(73) Patentinhaber: Tracoe Medical GmbH, 55268 Nieder-Olm (DE)
(72) Erfinder: Schnell, Ralf, 63500 Seligenstadt (DE); Altenburger, Jörg, 61231 Bad Nauheim (DE)
(74) Vertreter: WSL Patentanwälte Partnerschaft mbB

(56) Entgegenhaltungen:
- WO-A1-2006/089961
- DE-A1-102005 015 045
- MATSUBA KOHEI ET AL: "Cuff leakage caused by automatic cuff pressure monitor and water condensation in the inflation tube of Microcuff endotracheal tube.", ANAESTHESIA AND INTENSIVE CARE MAY 2019, Bd. 47, Nr. 3, Mai 2019 (2019-05), Seiten 300-301, XP009518191, ISSN: 0310-057X

## Beschreibung

Die vorliegende Erfindung betrifft eine Beatmungsvorrichtung, insbesondere einen Endotrachealtubus oder eine Tracheostomiekanüle, mit einem Kanülenrohr und einem dicht mit der Außenseite des Kanülenrohrs verbundenen Cuff, welcher über einen Füllschlauch mit einem Kontrollballon in Fluidverbindung steht oder in Verbindung gebracht werden kann.

Beatmungsvorrichtungen werden in die Trachea eines Patienten eingeführt, um den Patienten mit Luft bzw. Atemgas zu versorgen. Das Kanülenrohr kann durch eine im Hals eines Patienten unterhalb des Kehlkopfbereichs künstlich erzeugte Öffnung (Tracheostoma) oder über den Mund und Rachenraum in die Trachea eines Patienten eingebracht werden. Das Kanülenrohr dient mit seinem zentralen Lumen der Hindurchleitung von Luft bzw. Atemgas.

Der befüllbare Cuff auf dem äußeren Umfang des Kanülenrohrs dient der Abdichtung der Trachea in dem Volumen außerhalb des Kanülenrohrs. Ein solcher Cuff besteht üblicherweise aus einem aufblasbaren, flexiblen Schlauchabschnitt mit einer sehr geringen Wandstärke, dessen Endabschnitte außen auf dem Kanülenrohr anliegen und dort abgedichtet fixiert sind. Der Cuff hat mindestens in einem Abschnitt zwischen seinen Enden gegenüber dem Kanülenrohr ein deutliches Übermaß oder ist auf ein solches Übermaß aufblasbar. Beim Einführen des Kanülenrohrs in die Trachea liegt der zunächst ungefüllte Cuff eng an der Außenseite des Kanülenrohrs an und wird nach dem Einführen mit geringem Druck aufgeblasen, bis er mit der Wand der Trachea entlang seines Umfangs abdichtend, jedoch mit möglichst geringem Druck in Kontakt kommt.

Der geringe Druck soll verhindern, dass der Cuff mit einem Anpressdruck an der Wand der Trachea anliegt, der die Durchblutung der Trachealwand unterbrechen kann und bei längerer Dauer der Anlage die Trachealwand nachhaltig beschädigen könnte. Der Anpressdruck an der Trachealwand ist von dem Druck im Cuff zu unterscheiden, der unter Umständen auch den Cuff elastisch dehnen muss. Der minimale Druck im Cuff, ab dem eine ausreichende Abdichtung gegenüber der Trachea erreicht wird, hängt daher unter anderem von der Dicke und Flexibilität der Cufffolie ab.

Um eine bessere Kontrolle über den Anpressdruck des Cuffs an der Trachealwand zu haben, werden häufig sogenannte "High Volume Low Pressure Cuffs" (HVLP), verwendet, die von vornherein ein Übermaß aufweisen, so dass ihr maximales Volumen im nahezu drucklos aufgeblasenen Zustand das in der Trachea einzunehmende Volumen deutlich übersteigt.

Auf diese Weise muss sich das Material des Cuffs bei der vorgesehenen Verwendung nicht ausdehnen, sondern muss sich lediglich aus einem an dem Kanülenrohr eng anliegenden Zustand entfalten und an die Trachealwand an anschmiegen. Wegen des Übermaßes hat der Cuff aber auch in seinem an der Trachealwand anliegenden Zustand noch immer Falten. Damit der Cuff trotz solcher Falten eine ausreichende Abdichtung gegenüber der Trachea erreicht, sollte der Druck in einem "High Volume Low Pressure Cuff" etwa 15 hPa bis 30 hPa betragen. Dies ist dann auch der Druck, mit welchem der Cuff an der Wand der Trachea anliegt und den es einzuhalten und zu kontrollieren gilt. Solche Cuffs sind vorzugsweise sehr dünnwandig, um den Faltenquerschnitt soweit wie möglich zu reduzieren und um so zu vermeiden, dass Flüssigkeiten oder Sekrete entlang der Falten den Cuff passieren können und Komplikationen verursachen.

Zur Vermeidung von Trachealschäden einerseits und zur Erzielung einer ausreichenden Abdichtung andererseits ist es daher erforderlich, den Cuffdruck exakt einzustellen und regelmäßig zu überprüfen. Das gilt sowohl für "High Volume Low Pressure Cuffs" als auch für alle übrigen Arten von Cuffs, bei welchen der Druck, der im Cuff bzw. in einem mit dem Cuff in Fluidverbindung stehenden Volumen gemessen wird, um den zur Ausdehnung des Cuffs gegebenenfalls erforderlichen elastischen Anteil zu korrigieren ist. Dies hat allerdings den Nachteil, dass der so ermittelte Druckwert fehlerhaft ist, insbesondere wenn die elastische Rückstellkraft des Cuffmaterials im Laufe der Zeit nachlässt.

"High Volume Low Pressure Cuffs" haben dagegen den Vorteil, dass der im Cuff gemessene Druck nahezu exakt dem Druck entspricht, mit dem der Cuff auf die Trachealwand drückt.

Zum Einstellen und Überprüfen des Cuffsdrucks wird ein entsprechend konfektioniertes Manometer an einen Kontrollballon oder den Füllschlauch angeschlossen.

Allerdings kann sich unter Umständen in dem Cuff und/oder in dem Füllschlauch und/oder in dem Kontrollballon Kondenswasser ansammeln. Wassermoleküle können beispielsweise durch die dünne Cuffwand in das Innere des Cuffs diffundieren und aufgrund von Temperaturschwankungen kondensieren. Bei der Kontrolle oder Einstellung des Cuffdrucks kann das Kondenswasser auch in den Füllschlauch und/oder in den Kontrollballon gelangen.

Insbesondere in dem Füllschlauch befindliches Wasser kann zu Artefakten bei der Einstellung und Messung des Cuffdrucks führen. Wegen des geringen Querschnitts eines Füllschlauches und der im Vergleich zu Gasen relativ großen Viskosität und Benetzungsfähigkeit von Wasser wird das Wasser bei geringen Druckdifferenzen nicht durch den Füllschlauch hindurch gedrückt, sondern bildet eine Barriere in dem Füllschlauch und verfälscht so die Messung des Cuffdrucks. Eine Verfälschung des Messergebnisses ist insbesondere zu beobachten, wenn eine Wasserssäule in dem Füllschlauch durch Lufteinschlüsse unterbrochen ist.

Die Ansammlung von Wasser durch Eindiffundieren und damit auch die Entstehung von entsprechenden Artefakten könnte durch die Verwendung von Cufffolien mit größeren Wandstärken verhindert werden, da die Barriereeigenschaften gegen Wasser mit der Wandstärke ansteigen. Dem steht aber die Faltenbildung und die dadurch bedingte Beeinträchtigung einer sicheren Abdichtung bei geringen Cuffdrücken entgegen.

Aus der WO 2006/089961 A1 ist ein Cuff mit einer Beschichtung bekannt, die verhindern soll, dass Luftfeuchtigkeit in den Cuff eindringt. Allerdings schmiegen sich Cufffolien mit größeren Wandstärken und Beschichtung schlechter an die Trachealwand an und dichten daher die Trachea bei gegebenen Anpressdruck weniger effektiv ab (siehe z.B. EP 1 061 984 B2). Alternativ könnten für den Cuff selbst Materialen verwendet werden, die ein Eindiffundieren von Luftfeuchtigkeit in den Cuff erschweren. Gute Barriereeigenschaften gegenüber Luftfeuchtigkeit haben beispielsweise Polypropylen, Polyethylen oder Teflon. Diese lassen sich jedoch bei der Herstellung nicht sonderlich gut mit einem Kanülenrohr verkleben, sodass in der Praxis Folien aus Polyvinylchlorid oder Polyurethan ungeachtet der geringen Barriereeigenschaften gegenüber Wasser bevorzugt sind.

Demgegenüber besteht eine Aufgabe der vorliegenden Erfindung darin, eine Beatmungsvorrichtung zu schaffen, die einerseits einen flexiblen und sich gut an die Trachealwand anlegenden Cuff aufweist, gleichzeitig aber wasserbedingte Artefakte bei der Messung und Einstellung eines Cuffdrucks einer Beatmungsvorrichtung vermeidet oder zumindest reduziert.

Erfindungsgemäß wird diese Aufgabe durch eine Beatmungsvorrichtung der eingangs genannten Art gelöst, bei welcher das Innere des Cuffs und/oder des Füllschlauchs und/oder des Kontrollballons mindestens eine oberflächenaktive Substanz zur Herabsetzung der Oberflächenspannung von Wasser enthält.

Die oberflächenaktive Substanz reduziert bei Kontakt mit Wasser dessen Oberflächenspannung und bewirkt so, dass das Wasser mit geringerer Reibung und Haftung an der Wand des Füllschlauches leichter und bei geringen Druckunterschieden durch den Füllschlauch bewegt werden kann. Das Wasser mit herabgesetzter Oberflächenspannung bildet somit keine bzw. nur geringfügige Barrieren, die eine Messung und Einstellung des Cuffdrucks verfälschen könnten.

Die oberflächenaktiven Substanzen zur Herabsetzung der Oberflächenspannung von Wasser ermöglichen, den Innendurchmesser des Füllschlauchs gegenüber herkömmlichen Füllschläuchen deutlich zu reduzieren, ohne das der verringerte Innendurchmesser wasserbedingte Artefakte bei der Messung und Einstellung eines Cuffdrucks erzeugt. In einer Ausführungsform ist der Innendurchmesser des Füllschlauchs kleiner oder gleich 0,7 mm, vorzugsweise kleiner oder gleich 0,6 mm. Der Innendurchmesser des Füllschlauchs kann kleiner oder gleich 0,5 mm sein. Vorzugsweise ist der Innendurchmesser des Füllschlauchs größer oder gleich 0,3 mm.

Da der Füllschlauch meist in die Wand des Kanülenrohrs eingelassen oder außenliegend auf der Wand des Kanülenrohrs angeordnet ist, kann die Wand des Kanülenrohrs bei der Verwendung eines Füllschlauchs mit einem kleinen Innendurchmesser dünner ausgeführt werden. Der Innendurchmesser des Kanülenrohrs kann um den durch die Verwendung eines Füllschlauchs mit kleinem Innendurchmesser eingesparten Platzbedarf vergrößert werden. Ein Kanülenrohr mit einem vergrößerten Innendurchmesser erzeugt einen geringeren Atemwiderstand als ein Kanülenrohr mit einem im Vergleich kleineren Innendurchmesser. Die Verwendung von Tensiden und einem Füllschlauch mit einem Innendurchmesser von kleiner oder gleich 0,7 mm ermöglicht es, eine Beatmungsvorrichtung mit einem reduzierten Atemwiderstand bereitzustellen.

In einer Ausführungsform ist die oberflächenaktive Substanz zur Herabsetzung der Oberflächenspannung von Wasser in einer Menge von mindestens 0,01 g und/oder nicht mehr als 0,3 g, vorzugsweise nicht mehr als 0,1 g, vorhanden.

Die oberflächenaktive Substanz zur Herabsetzung der Oberflächenspannung von Wasser kann mindestens ein wasserlösliches Tensid enthalten oder aus einem wasserlöslichen Tensid oder aus mehreren wasserlöslichen Tensiden bestehen. Das mindestens eine wasserlösliche Tensid oder die mehreren wasserlöslichen Tenside sind vorzugsweise ausgewählt aus der Gruppe umfassend: lineare Alkylbenzosulfonate (LAS), Alkylpolygglycoside (APG), Esterquats (EQ), Fettalkoholethoxylate (FAEO), Fettalkoholsulfate (FAS), Fettalkoholethersulfate (FES), Tyloxapol, Polysorbat 80, Natrium- oder Kaliumsalze von Fettsäuren, Emulgatoren, Natrium-, Kalium-, und CalciumSalze von Speisefettsäuren (E 470a), Mono- und Diglyceride von Speseefettsäuren (E 471), Polyoxyethylensorbitanmonolaurat (E 432), Polyoxyethylensorbitanmonooleat (E 433), Polyoxyethylensorbitanmonopalmitat (E 434), Polyoxyethenlyensorbitanmomostearat (E 435), Polyoxyethylen20-Sorbitantri-Stearat (E 436) und Propylenglycolalginat (E 405).

Die oberflächenaktive Substanz kann zweckmäßigerweise dort in die Beatmungsvorrichtung eingebracht werden, wo sie mit dem Kondenswasser in Kontakt kommen kann. Beispielsweise kann die oberflächenaktive Substanz in das Innere des Cuffs und/oder in das Innere des Füllschlauchs und/oder in das Innere des Kontrollballons eingebracht werden. Grundsätzlich ist es ausreichend, wenn die mindestens eine oberflächenaktive Substanz an einer Stelle der Beatmungsvorrichtung vorhanden ist. Sobald die oberflächenaktive Substanz mit Wasser in Verbindung kommt, so löst und verteilt sich diese mindestens eine oberflächenaktive Substanz in dem Wasser.

In einer Ausführungsform liegt die mindestens eine oberflächenaktive Substanz als eine Lösung, eine wässrige Lösung, als Schaum oder als ein löslicher Feststoff fließfähig vor.

Durch Ihre Fließfähigkeit ist die mindestens eine oberflächenaktive Substanz selbst bewegbar und damit erhöht sich die Wahrscheinlichkeit, dass die mindestens eine oberflächenaktive Substanz mit dem Wasser in dem Cuff der Beatmungsvorrichtung in Kontakt kommt.

Eine Lösung, eine wässrige Lösung, ein Schaum oder ein löslicher Feststoff, beispielsweise in Form eines Pulvers, kann durch die Luft in dem Füllschlauch, dem Kontrollballon oder dem Cuff verwirbelt und verteilt werden. Die mindestens eine oberflächenaktive Substanz kann auch in Form einer löslichen Beschichtung die Innenfläche des Cuffs und/oder die Innenfläche des Füllschlauchs und/oder die Innenfläche des Kontrollballons teilweise oder ganz auskleiden.

Die erfindungsgemäße Aufgabe wird auch durch ein Verfahren zur Erleichterung eines Wassertransports durch einen Füllschlauch einer Beatmungsvorrichtung, insbesondere Endotrachealtubus oder Tracheostomiekanüle, gelöst, wobei die Beatmungsvorrichtung ein Kanülenrohr und ein dicht mit der Außenseite des Kanülenrohrs verbundenen Cuff aufweist, der über den Füllschlauch mit einem Kontrollballon in fluider Kommunikation verbunden ist, wobei das Verfahren den Schritt aufweist:
Einbringen mindestens einer oberflächenaktiven Substanz zur Herabsetzung der Oberflächenspannung von Wasser in das Innere des Cuffs und/oder des Füllschlauchs und/oder des Kontrollballons.

Die mindestens eine oberflächenaktive Substanz kann bei der Herstellung der Beatmungsvorrichtung oder nach der Herstellung der Beatmungsvorrichtung eingebracht werden. Beispielsweise kann die oberflächenaktive Substanz als wässrige Lösung in den Kontrollballon und/oder den Cuff und/oder den Füllschlauch und/oder über ein am Kontrollballon befindliches Füllventil injiziert werden. Aufgrund der geringen Barriereeigenschaft des Cuffs gegenüber Wasser verdunstet das Waser und die oberflächenaktive Substanz bleibt feinverteilt im Inneren des Kontrollballons und/oder des Füllschlauchs und/oder des Cuffs zurück. Im Unterschied zu Waser können die oberflächenaktiven Substanzen aufgrund ihrer deutlich erhöhten Molekülgröße nicht durch die Wand des Cuffs nach Außen diffundieren.

Weitere Vorteile, Merkmale und Anwendungsmöglichkeiten der vorliegenden Erfindung werden anhand der folgenden Beschreibung einer Ausführungsform und den dazugehörigen Figuren deutlich, wobei gleiche Bezugszeichen auf gleiche Elemente verweisen. Es zeigen:
- Figur 1:: eine Beatmungsvorrichtung gemäß einer Ausführungsform der vorliegenden Erfindung, und
- Figur 2:: einen vergrößerten Längsschnitt durch den Füllschlauchs aus Figur 1.

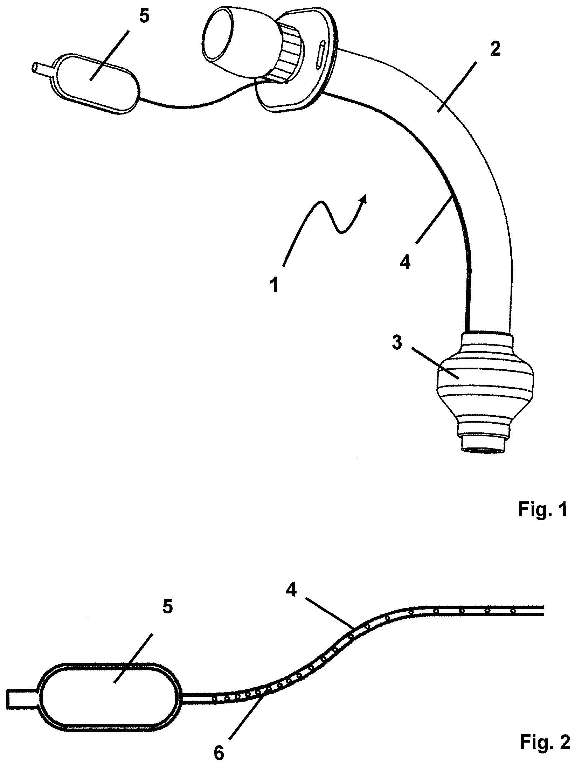

In der Figur 1 ist eine Beatmungsvorrichtung gemäß einer Ausführungsform der vorliegenden Erfindung gezeigt. Die Beatmungsvorrichtung 1 ist eine Tracheostomiekanüle mit einem Kanülenrohr 2 und einem dicht mit der Außenseite des Kanülenrohrs 2 verbundenen Cuffs 3. Der Cuff 3 ist in fluider Kommunikation über einen Füllschlauch 4 mit einem Kontrollballon 5 verbunden. Über den Füllschlauch 4 und den Kontrollballon 5 kann der Cuff 3 mit einem Gas befüllt bzw. der Cuffdruck reguliert werden.

Wie in der Figur 2, die einen vergrößerten Längsschnitt durch den Füllschlauch 4 und den Kontrollballon 5 zeigt, angedeutet, enthält das innere des Füllschlauchs 4 mindestens eine oberflächenaktive Substanz 6 zur Herabsetzung der Oberflächenspannung von Wasser. Die mindestens eine oberflächenaktive Substanz 6 wurde in Form eines löslichen Pulvers bei der Herstellung der Beatmungsvorrichtung 1 in den Füllschlauch 4 eingebracht. Die mindestens eine oberflächenaktive Substanz liegt bei dieser Ausführungsform in einer Menge von z. B. 0,01 g bis 0,1 g vor.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarerer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschreiben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht. Die Erfindung ist beschränkt durch seine Ansprüche und soll im Rahmen der Beschreibung gestützt werden.

### Bezugszeichenliste

- 1: Beatmungsvorrichtung
- 2: Kanülenrohr
- 3: Cuff
- 4: Füllschlauch
- 5: Kontrollballon
- 6: oberflächenaktive Substanz

## Patentansprüche

1. Beatmungsvorrichtung (1), insbesondere Endotrachealtubus oder Tracheostomiekanüle, mit einem Kanülenrohr (2) und einem dicht mit der Außenseite des Kanülenrohrs (2) verbundenen Cuff (3), welcher über einen Füllschlauch (4) mit einem Kontrollballon (5) in Fluidverbindung steht oder in Fluidverbindung gebracht werden kann.
**dadurch gekennzeichnet, dass**
das Innere des Cuffs (3) und/oder das Innere des Füllschlauchs (4) und/oder das Innere des Kontrollballons (5) mindestens eine oberflächenaktive Substanz (6) zur Herabsetzung der Oberflächenspannung von Wasser enthält.

2. Beatmungsvorrichtung (1) nach Anspruch 1, **dadurch gekennzeichnet, dass** die oberflächenaktive Substanz (6) zur Herabsetzung der Oberflächenspannung von Wasser in einer Menge von mindestens 0,01 g und/oder nicht mehr als 0,3 g, vorzugsweise nicht mehr als 0,1 g, vorhanden ist.

3. Beatmungsvorrichtung (1) nach Anspruch 1 oder 2, **dadurch gekennzeichnet, dass** die oberflächenaktive Substanz (6) zur Herabsetzung der Oberflächenspannung von Wasser mindestens ein Tensid enthält oder aus einem Tensid oder mehreren Tensiden besteht.

4. Beatmungsvorrichtung (1) nach einem der Ansprüche 1 bis 3, **dadurch gekennzeichnet, dass** die mindestens eine oberflächenaktive Substanz (6) in Form einer Lösung, einer wässrigen Lösung, eines Schaums oder eines löslichen Feststoffs vorliegt.

5. Beatmungsvorrichtung (1) nach einem der Ansprüche 1 bis 4, **dadurch gekennzeichnet, dass** der Füllschlauch einen Innendurchmesser von kleiner 0,7 mm, vorzugsweise kleiner oder gleich 0,6 mm, wobei vorzugsweise der Innendurchmesser größer oder gleich 0,3 mm ist.

6. Verfahren zur Verringerung des Durchflusswiderstandes von Wasser in einem Füllschlauch (4) einer Beatmungsvorrichtung (1), insbesondere Endotrachealtubus oder Tracheostomiekanüle, wobei die Beatmungsvorrichtung (1) ein Kanülenrohr (2) und ein dicht mit der Außenseite des Kanülenrohrs (2) verbundenen Cuff (3) aufweist, der über den Füllschlauch (4) mit einem Kontrollballon (5) in Fluidverbindung steht oder bringbar ist, **gekennzeichnet durch**
a) Einbringen mindestens einer oberflächenaktiven Substanz (6) zur Herabsetzung der Oberflächenspannung von Wasser in das Innere des Cuffs (3) und/oder in das Innere des Füllschlauchs (4) und/oder des Kontrollballons (5).

7. Verfahren nach Anspruch 6, **dadurch gekennzeichnet, dass** der Schritt a) bei der Herstellung der Beatmungsvorrichtung (1) oder nach der Herstellung der Beatmungsvorrichtung (1) ausgeführt wird.

8. Verfahren nach Anspruch 6 oder 7, **dadurch gekennzeichnet, dass** die Menge der mindestens einen oberflächenaktiv Substanz (6) zur Herabsetzung der Oberflächenspannung von Wasser in einer fließfähigen Form als Lösung, insbesondere als wässrige Lösung, als Schaum oder als lösliches Pulver eingebracht wird.

9. Verfahren nach einem der Ansprüche 6 bis 8, **gekennzeichnet durch** b) Injizieren der mindestens einen oberflächenaktiven Substanz (6) zur Herabsetzung der Oberflächenspannung von Wasser in Form einer wässrigen Lösung in den Cuff (3) und/oder in den Kontrollballon (4) und/oder in den Füllschlauch (5), und c) Verdunsten des Wasseranteils der wässrigen Lösung.

10. Verfahren nach einem der Ansprüche 6 bis 9, **dadurch gekennzeichnet, dass** die oberflächenaktive Substanz (6) zur Herabsetzung der Oberflächenspannung von Wasser in einer Menge von mindestens 0,01 g und/oder nicht mehr als 0,3 g, vorzugsweise nicht mehr als 0,1 g, eingebracht wird.

## Claims

1. Ventilation apparatus (1), in particular endotracheal tube or tracheostomy tube, with a cannula tube (2) and a cuff (3), tightly connected to the outside of the cannula tube (2), which can be in fluid connection or brought into fluid connection with a pilot balloon (5) via a filling hose (4),
**characterized in that**
the inside of the cuff (3) and/or the inside of the filling hose (4) and/or the inside of the pilot balloon (5) contains at least one surface-active substance (6) for reducing the surface tension of water.

2. Ventilation apparatus (1) according to claim 1, **characterized in that** the surface-active substance (6) for reducing the surface tension of water is present in a quantity of at least 0.01 g and/or no more than 0.3 g, preferably no more than 0.1 g.

3. Ventilation apparatus (1) according to claim 1 or 2, **characterized in that** the surface-active substance (6) for reducing the surface tension of water contains at least one surfactant or consists of one surfactant or several surfactants.

4. Ventilation apparatus (1) according to one of claims 1 to 3, **characterized in that** the at least one surface-active substance (6) is present in the form of a solution, an aqueous solution, a foam or a soluble solid.

5. Ventilation apparatus (1) according to one of claims 1 to 4, **characterized in that** the filling hose [has] an inside diameter of less than 0.7 mm, preferably less than or equal to 0.6 mm, wherein preferably the inside diameter is greater than or equal to 0.3 mm.

6. Method for reducing the flow resistance of water in a filling hose (4) of a ventilation apparatus (1), in particular endotracheal tube or tracheostomy tube, wherein the ventilation apparatus (1) has a cannula tube (2) and a cuff (3), tightly connected to the outside of the cannula tube (2), which is in or can be brought into fluid connection with a pilot balloon (5) via the filling hose (4), **characterized by**
a) introducing at least one surface-active substance (6) for reducing the surface tension of water into the inside of the cuff (3) and/or into the inside of the filling hose (4) and/or the pilot balloon (5).

7. Method according to claim 6, **characterized in that** step a) is carried out during the production of the ventilation apparatus (1) or after the production of the ventilation apparatus (1).

8. Method according to claim 6 or 7, **characterized in that** the quantity of the at least one surface-active substance (6) for reducing the surface tension of water is introduced in a flowable form as a solution, in particular as an aqueous solution, as a foam or as a soluble powder.

9. Method according to one of claims 6 to 8, **characterized by** b) injecting the at least one surface-active substance (6) for reducing the surface tension of water into the cuff (3) and/or into the pilot balloon (4) and/or into the filling hose (5) in the form of an aqueous solution, and c) evaporating the water content of the aqueous solution.

10. Method according to one of claims 6 to 9, **characterized in that** the surface-active substance (6) for reducing the surface tension of water is introduced in a quantity of at least 0.01 g and/or no more than 0.3 g, preferably no more than 0.1 g.

## Revendications

1. Dispositif de respiration (1), notamment tube endotrachéal ou canule de trachéotomie, avec un tube de canule (2) et un coussinet (3) relié de façon étanche à la face extérieure du tube de canule (2), qui est en communication fluidique ou qui peut être mis en communication fluidique, par un tube de remplissage (4), avec un ballonnet de contrôle (5),
**caractérisé en ce que**
l'intérieur du coussinet (3) et/ou l'intérieur du tube de remplissage (4) et/ou l'intérieur du ballonnet de contrôle (5) contient au moins une substance tensioactive (6) destinée à baisser la tension superficielle d'eau.

2. Dispositif de respiration (1) selon la revendication 1, **caractérisé en ce que** la substance tensioactive (6) destinée à baisser la tension superficielle d'eau est présente en une quantité d'au moins 0,01 g et/ou de pas plus que 0,3 g, de préférence de pas plus que 0,1 g.

3. Dispositif de respiration (1) selon la revendication 1 ou 2, **caractérisé en ce que** la substance tensioactive (6) destinée à baisser la tension superficielle d'eau contient au moins un tensioactif ou est constituée d'un tensioactif ou de plusieurs tensioactifs.

4. Dispositif de respiration (1) selon l'une des revendications 1 à 3, **caractérisé en ce que** la substance tensioactive (6) destinée à baisser la tension superficielle d'eau se présente sous la forme d'une solution, d'une solution aqueuse, d'une mousse ou d'une substance solide soluble.

5. Dispositif de respiration (1) selon l'une des revendications 1 à 4, **caractérisé en ce que** le tube de remplissage a un diamètre intérieur de moins de 0,7 mm, de préférence moins de ou égal à 0,6 mm, le diamètre intérieur étant de préférence supérieur à ou égal à 0,3 mm.

6. Procédé de réduction de la résistance au flux d'eau dans un tube de remplissage (4) d'un dispositif de respiration (1), notamment tube endotrachéal ou canule de trachéotomie, un tube de canule (2) et un coussinet (3) relié de façon étanche à la face extérieure du tube de canule (2), qui est en communication fluidique ou qui peut être mis en communication fluidique, par un tube de remplissage (4), avec un ballonnet de contrôle (5), **caractérisé par**
a) l'introduction d'au moins une substance tensioactive (6) destinée à baisser la tension superficielle d'eau à l'intérieur du coussinet (3) et/ou à l'intérieur du tube de remplissage (4) et/ou du ballonnet de contrôle (5).

7. Procédé selon la revendication 6, **caractérisé en ce que** l'étape a) est mise en oeuvre lors de la fabrication du dispositif de respiration (1) ou après la fabrication du dispositif de respiration (1).

8. Procédé selon la revendication 6 ou 7, **caractérisé en ce que** la quantité de ladite au moins une substance tensioactive (6) destinée à baisser la tension superficielle d'eau est introduite sous une forme fluide comme solution, notamment comme solution aqueuse, comme mousse ou comme poudre soluble.

9. Procédé selon l'une des revendications 6 à 8, **caractérisé par** b) une injection de ladite au moins une substance tensioactive (6) destinée à baisser la tension superficielle d'eau sous la forme d'une solution aqueuse dans le coussinet (3) et/ou dans le ballonnet de contrôle (4) et/ou dans le tube de remplissage (5) et c) évaporation de la proportion d'eau de la solution aqueuse.

10. Procédé selon l'une des revendications 6 à 9, **caractérisé en ce que** la substance tensioactive (6) destinée à baisser la tension superficielle d'eau est introduite en une quantité d'au moins 0,01 g et/ou de pas plus que 0,3 g, de préférence de pas plus que 0,1 g.
